Europäisches Patentamt

European Patent Office

Office européen des brevets

(19)

(11) Veröffentlichungsnummer: **0 135 140**
**A1**

(12) # EUROPÄISCHE PATENTANMELDUNG

(21) Anmeldenummer: **84109648.0**

(22) Anmeldetag: **14.08.84**

(51) Int. Cl.⁴: **A 61 M 25/00**

(30) Priorität: **20.08.83 DE 3330149**

(43) Veröffentlichungstag der Anmeldung:
**27.03.85 Patentblatt 85/13**

(84) Benannte Vertragsstaaten:
**CH FR GB IT LI NL**

(71) Anmelder: **Aero-Pump GmbH, Zerstäuberpumpen**
**Otto-Schwabe-Strasse 4**
**D-6203 Hochheim/Main(DE)**

(72) Erfinder: **Schwab, Egon**
**Otto-Schwabe-Strasse 4**
**D-6203 Hochheim(DE)**

(72) Erfinder: **Padar, Steve**
**Theresenstrasse 17**
**D-6235 Kelkheim(DE)**

(74) Vertreter: **Knoblauch, Ulrich, Dr.-Ing.**
**Kühhornshofweg 10**
**D-6000 Frankfurt am Main 1(DE)**

(54) **Rückschlagventil für medizinische Zwecke, insbesondere für Ballonkatheter.**

(57) Ein Rückschlagventil für medizinische Zwecke, insbesondere für Ballonkatheter, weist ein hohles Ventilgehäuse (3) auf, das an einer Stirnseite eine Anschlußöffnung (8) zum Einführen eines Spritzen-Mundstückes und zwei Anschläge (16, 17) für einen Schieber (18) aufweist. Dieser übersteuert mit seiner Umfangsfläche mindestens eine Ventilöffnung (15) am zylindrischen Innenumfang (14) des Gehäuses (3). Der erste Anschlag (16) hat eine so geringe Erhebung, daß der Schieber (18) unter Ausnutzung der elastischen Verformbarkeit von Ventilgehäuse (3) und/oder Schieber durch die Anschlußöffnung (8) hindurch einführbar ist. Dies ermöglicht es, den zweiten Anschlag (17) an der der Anschlußöffnung (8) gegenüberliegende Stirnseite schon bei der Herstellung fertig zu formen.

Fig.1

- 4 -

A 112

AERO Pump GmbH, Zerstäuberpumpen, 6203 Hochheim

Rückschlagventil für medizinische Zwecke, insbesondere
für Ballonkatheter

Die Erfindung bezieht sich auf ein Rückschlagventil
für medizinische Zwecke, insbesondere für Ballonkatheter, bei dem ein hohles Ventilgehäuse an einer Stirnseite eine Anschlußöffnung zum Einführen eines Spritzen-
Mundstückes, an der anderen Stirnseite mindestens eine
Durchgangsöffnung und in seinem Innenraum nahe der Anschlußöffnung einen ersten Anschlag und, zur gegenüberliegenden Stirnseite hin versetzt, einen zweiten Anschlag
für ein Verschlußstück aufweist und das Verschlußstück
vom Spritzen-Mundstück gegen eine elastische Kraft aus
einer die stirnseitigen Öffnungen gegeneinander sperrenden Schließstellung in eine diese Öffnungen untereinander
verbindende Öffnungsstellung verlagerbar ist.

Bei einer bekannten Rückschlagventil dieser Art
(US-PS 38 31 629) dient der erste Anschlag als Ventilsitz, an dem sich eine Schulter des Verschlußstücks
in der Schließstellung anlegt. Zum Zweck der Führung
im Innenraum des Ventilgehäuses ist das Verschlußstück
außen mit Axialrippen versehen. Das hintere Ende des Verschlußstücks stützt sich am zweiten Anschlag ab und kann in Axialrichtung elastisch verformt werden. Der zweite Anschlag entsteht nach dem Einsetzen des Verschlußstücks in den Innenraum des Ventilgehäuses dadurch, daß ein einstückig mit dem Gehäuse gebildeter zylindrischer Rand nachträglich nach innen umgebördelt wird.

Dieses Umbördeln stellt einen zusätzlichen Arbeitsgang dar, der die Herstellung erschwert. Er beschränkt auch die Zahl der brauchbaren Kunststoffe zur Herstellung des Ventilgehäuses erheblich. Ferner bereitet es Schwierigkeiten, die Umbördelung so vorzunehmen, daß im Ruhezustand das Verschlußstück mit einem vorgegebenen Axialdruck gegen den Sitz gedrückt wird.

Der Erfindung liegt die Aufgabe zugrunde, ein Rückschlagventil der eingangs beschriebenen Art anzugeben, bei dessen Herstellung zur Bildung des zweiten Anschlages kein zusätzlicher Arbeitsgang erforderlich ist.

Diese Aufgabe wird erfindungsgemäß dadurch gelöst, daß der Anschlag am Ventilgehäuse angeformt ist, daß das Verschlußstück ein Schieber ist, der mit seiner Umfangsfläche mindestens eine Ventilöffnung am zylindrischen Innenumfang des Gehäuses übersteuert und daß der erste Anschlag eine so geringe Erhebung hat, daß der Schieber unter Ausnutzung der elastischen Verformbarkeit von Ventilgehäuse und/oder Schieber durch die Anschlußöffnung hindurch einführbar ist.

Wegen der Ausbildung als Schieberventil wird der erste Anschlag nicht mehr als Ventilsitz benötigt. Er muß lediglich das Herausfallen des Verschlußstücks verhindern; hierfür genügen verhältnismäßig kleine Flächen am ersten Anschlag. Infolgedessen kann der erste Anschlag mit einer so geringen Erhebung ausgeführt werden, daß das Verschlußstück bei der Montage mit nur geringfügig erhöhtem Kraftaufwand an Ort und Stelle gebracht werden kann. Der zweite Anschlag kann daher beim Spritzen des Ventilgehäuses in seiner endgültigen Gestalt angeformt werden, da das Einführen des Verschlußstückes von der Seite des ersten Anschlages und nicht von der Seite des zweiten Anschlages her erfolgt. Ein weiterer Vorteil des Schieberventils liegt darin, daß die Dichtkraft nicht von axialen elastischen Kräften abhängt, sondern durch die Abmessungen von Ventilgehäuse und Verschluß- stück am Innen- bzw. Außenumfang vorgegeben sind. Ein Schieber hat auch ohne Zusatzmaßnahmen eine gute Führung im Gehäuse, insbesondere wenn er eine entsprechende Länge hat.

Günstig ist es wenn, der zweite Anschlag von einer steifen, einstückig und gemeinsam mit dem Ventilgehäuse geformten Stirnwand getragen ist. Auf diese Weise wird das Ventilgehäuse an dem dem ersten Anschlag gegenüberliegenden Ende versteift. Es kann daher verhältnismäßig dünnwandig ausgebildet werden, was die elastische Aufwei- tung beim Einführen des Verschlußstücks erleichtert.

Insbesondere kann der erste Anschlag ein Ringwulst sein. Bei einem Ringwulst reicht eine sehr geringe Erhebung, um noch eine ausreichende Anschlagfläche zur Verfügung zu stellen.

Mit besonderem Vorteil weist der Schieber mindestens zwei in Axialrichtung gegeneinander versetzte Umfangs- Dichtlippen auf. Ein solcher Schieber läßt sich sehr einfach montieren, da die Dichtlippen leichter über den ersten Anschlag zu schieben sind als eine kontinuierliche Zylinderfläche.

Des weiteren empfiehlt es sich, daß der Schieber zumindest über einen Teil seiner axialen Länge einen mit der Durchgangsöffnung in Verbindung stehenden Hohlraum aufweist. Wenn das Rückschlagventil einen Druck abdichten soll, wirkt dieser auch im Hohlraum und unterstützt die radial nach außen wirkenden Dichtkräfte.

Zur Erzeugung der elastischen Kraft kann insbesondere eine Schraubenfeder, die sich im Bereich des zweiten Anschlags am Ventilgehäuse abstützt, an dem Schieber angreifen. Diese Schraubenfeder kann so bemessen werden, daß sie den Schieber in seine Ruhelage am ersten Anschlag zurückführt, wenn er nicht mehr vom Spritzen-Mundstück belastet ist.

Insbesondere kann sich die Schraubenfeder in den Hohlraum des Schiebers erstrecken. Die Feder kann daher verhältnismäßig lang ausgebildet sein, so daß sich die Federkraft während des Hubes des Schiebers verhältnismäßig wenig ändert.

Bei einer bevorzugten Ausführungsform ist dafür gesorgt, daß die mindestens eine Ventilöffnung die Umfangswand des Ventilgehäuses durchsetzt und mit einer von außen abgedeckten, auch die Durchgangsöffnung bildenden Nut am Außenumfang des Ventilgehäuses verbunden ist. Ein solches Ventilgehäuse läßt sich leicht formen. Die äußere Nut ist im einfachsten Fall durch das über das Ventilgehäuse geschobene Schlauchende abgedeckt.

Bei einer anderen Ausführungsform ist die mindestens eine Ventilöffnung durch eine Nut am Innenumfang des Ventilgehäuses, die eine größere axiale Erstreckung hat als die dichtende Länge des Schiebers, gebildet. Da die Strömungskanäle nur im Inneren des Gehäuses verlaufen, wird vermieden, daß bei zu starker Spannung des Schlauches oder durch ein nicht ganz einwandfreies Verkleben Störungen in einem äußeren Gehäusekanal auftreten.

Zweckmäßigerweise hat die mindestens eine Ventilöffnung einen größeren axialen Abstand vom ersten Anschlag als die dichtende Länge des Schiebers. In der Schließstellung befindet sich dann die gesamte dichtende Länge zwischen Anschlußöffnung und Ventilöffnung, so daß sich eine besonders gute Abdichtung ergibt.

Der Schieber kann mit sehr kleinem Durchmesser ausgeführt werden. Entsprechend klein ist dann auch der Durchmesser des Ventilgehäuses. Insbesondere sollte der Außenumfang des Ventilgehäuses zylindrisch sein und einen Durchmesser von 7mm haben. Ein solches Rückschlagventil kann ohne zusätzlichen Adapter für die gebräuchlichsten Schlauchgrößen verwendet werden, nämlich für einem Gummischlauch mit einem Innendurchmesser von ca. 6,0 mm und für einen Kunststoffschlauch mit einem Innendurchmesser von ca. 7,2 mm. Das Ventilgehäuse passt mit strammem Dehnungssitz in den Gummischlauch. Es passt andererseits mit geringem Spiel in einen Kunststoffschlauch, wo es entweder festgeklebt oder mit Hilfe einer Manschette befestigt werden kann.

Die Erfindung wird nachstehend anhand in der Zeichnung dargestellter, bevorzugter Ausführungsbeispiele näher erläutert. Es zeigen:

Fig. 1    einen Längsschnitt durch ein Rückschlagventil gemäß der Erfindung in der Schließstellung,

Fig. 2    das Rückschlagventil der Fig. 1 in der Öffnungsstellung,

Fig. 3    ein anderes Ausführungsbeispiel in der Schließstellung und

Fig. 4    das Ausführungsbeispiel der Fig. 3 in der
          Öffnungsstellung.

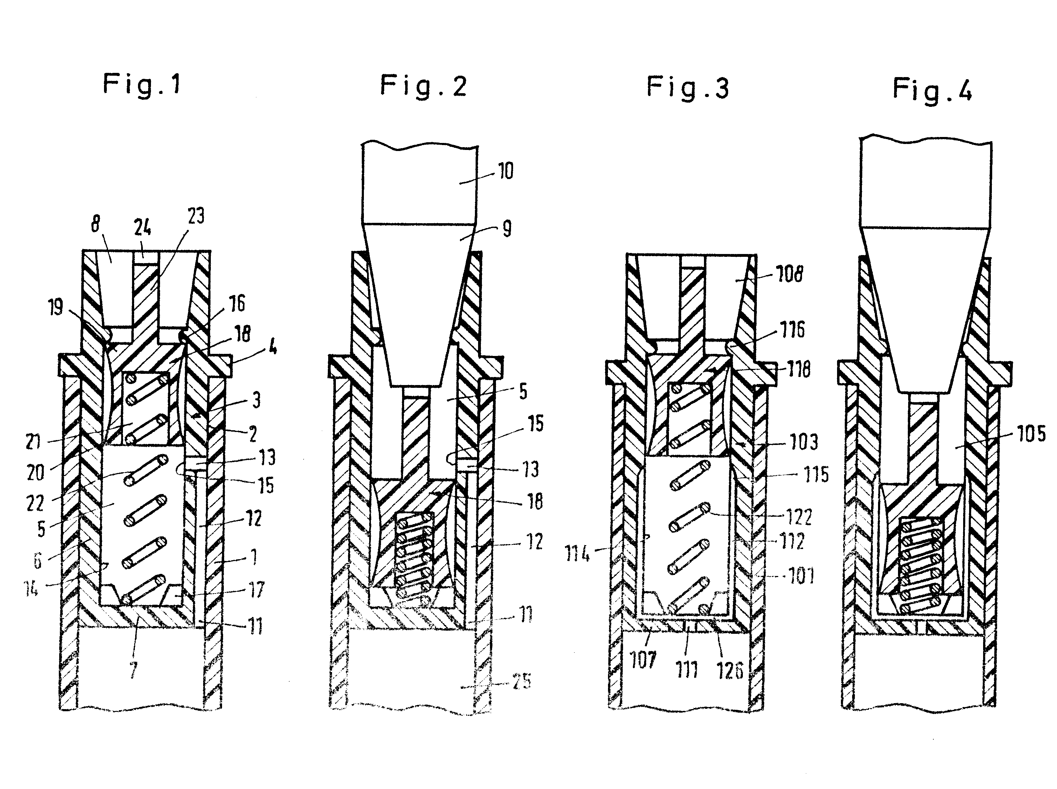

Bei der Ausführungsform nach Fig. 1 und 2 ist in das
Ende eines Schlauches 1, der beispielsweise zu dem aufblasbaren Ballon eines Katheters führt, auf den Außenumfang 2 eines Ventilgehäuses 3 bis zum Anschlag an einen
Flansch 4 geschoben. Der Schlauch 1 kann an dieser Stelle durch Eigenelastizität gehalten werden, beispielsweise wenn er aus Gummi oder einem anderen elastischen
Material besteht; er kann aber auch festgeklebt oder
durch eine zusätzliche Schelle, Manschette o.dgl. festgehalten werden. Das Ventilgehäuse 3 besitzt einen Innenraum 5, der von einer Umfangswand 6 umgeben und durch
eine starre, einstückig mit der Umfangswand 6 geformte
Stirnwand 7 abgeschlossen ist. Das Ventilgehäuse 3 besitzt an einer Stirnseite eine konische Anschlußöffnung
8 zum Einführen des konischen Mundstücks 9 einer Spritze
10 und an der gegenüberliegenden Seite eine Durchgangsöffnung 11, die durch das Ende einer axialen Außennut
12 in der Umfangswand 6 gebildet ist. Diese Außennut
12 steht über eine Radialbohrung 13 mit dem Innenraum
5 in Verbindung, wobei sich am Innenumfang 14 eine Ventilöffnung 15 ergibt. Nahe der Anschlußöffnung 8 ist
ein nach innen gerichteter erster Anschlag 16 in der
Form eines flachen Ringwulstes vorgesehen. Von der Stirnwand 7 ragen ein zweiter Anschlag 17 in der Form einzelner in Umfangsrichtung versetzter Blöcke nach oben.

Zwischen diesen beiden Anschlägen 16 und 17 ist ein
Schieber 18, der das Verschlußstück des Rückschlagventils bildet, verlagerbar. Der Schieber besitzt zwei
in Umfangsrichtung verlaufende Dichtlippen 19 und 20, die unter dem Einfluß von durch die Abmessungen des Schieber 18 mit Bezug auf das Ventilgehäuse 3 vorgegebenen Radialkräften dichtend an dem Innenumfang 14 des Ventilgehäuses 3 anliegen. Der Schieber 18 besitzt einen Hohlraum 21. In diesen greift eine Schraubenfeder 22, die sich zwischen den Blöcken des zweiten Anschlages 17 an der Stirnwand 7 abstützt und den Schieber 18 in die Schließstellung der Fig. 1 drückt. Zur mechanischen Kopplung mit dem Mundstück 9 weist der Schieber 18 einen nach außen ragenden Stift 23 mit einer stirnseitigen Quernut 24 auf. An diesem Stift kann, wie Fig. 2 zeigt, das Mundstück 9 angreifen und den gesamten Schieber 18 unter Zusammendrückung der Feder 22 gegen den zweiten Anschlag 17 drücken, so daß das Rückschlagventil seine Öffnungsstellung einnimmt.

In dieser Öffnungsstellung strömt Luft oder ein anderes Fluidum, das von der Spritze 10 geliefert wird, in den oberen Teil des Innenraums 5, weiter über die Ventilöffnung 15, die Radialbohrung 13, die äußere Nut 12 und die Durchgangsöffnung 11 in das Innere 25 des Schlauches 1 und damit zur Verbrauchs- oder Arbeitsstelle.

Wird das Mundstück 9 zurückgezogen, kehrt der Schieber 18 unter dem Einfluß der Feder 22 in die in Fig. 1 veranschaulichte Schließstellung zurück. In dieser Stellung liegen beide Dichtlippen 19 und 20 zwischen der Anschlußöffnung 8 und der Ventilöffnung 15, so daß der Innenraum 25 des Schlauches sicher gegenüber der Anschlußöffnung 8 abgedichtet ist. Hierbei wirkt der Druck im Schlauch 1 zusätzlich zur Feder 22, um den Schieber 18 in der veranschaulichten Schließlage zu halten. Die Feder 22 braucht daher in der gestreckten Stellung der Fig. 1 nur eine geringe Vorspannung zu haben. Sie kann deshalb verhältnismäßig schwach und billig hergestellt werden.

Der Druck wirkt auch im Hohlraum 21. Je höher dieser Druck ist, umso stärker sind die zumindest auf die Dichtlippe 20 wirkenden radialen Kräfte, so daß die Dichtwirkung sichergestellt ist.

Schieber 18 und Ventilgehäuse 3 werden aus Kunststoff gespritzt und dann automatisch montiert. Zu diesem Zweck brauchen lediglich die Feder 22 und der Schieber 18 in den Innenraum 5 eingeschoben zu werden. Hierbei läßt sich der Schieber 18 am ersten Anschlag 16 vorbeischieben, weil dieser sich so wenig über den Innenumfang 14 nach innen erhebt, daß die übliche Elastizität der Kunststoffe von Ventilgehäuse 3 und/oder Schieber 18 ausreicht, um die hierzu erforderliche Verformung zu bewirken. An die Kunststoffe werden keine hohen Ansprüche gestellt. Man kann daher eine Hochgeschwindigkeits-Spritzgußfertigung durchführen, so daß sich sehr niedrige Kosten ergeben. Als Kunststoffmaterial kommt beispielsweise ein Polyolefin o.dgl. in Betracht. Die Herstellung ist daher einfach und billig. Das Rückschlagventil kann daher als billiger Wegwerfartikel geliefert werden.

Bei der Ausführungsform nach den Fig. 3 und 4 werden für entsprechende Teile um 100 erhöhte Bezugszeichen benutzt. Unterschiedlich ist im wesentlichen, daß als Ventilöffnung 115 am Innenumfang 114 ausgebildete Axialnuten 112 vorgesehen sind, die sich auch noch als stirnseitige Nuten 126 fortsetzen können. Die Durchgangsöffnung 111 befindet sich in der Mitte der Stirnwand 107.

Wenn hier der Schieber 118 aus der Schließstellung der Fig. 3 in die Öffnungsstellung der Fig. 4 verlagert wird, strömt die Luft oder das Fluidum nur im Innenraum 105 des Ventilgehäuses 103. Die Axialnuten 112 können sich nicht von außen her zusetzen.

Das Rückschlagventil läßt sich für die verschiedensten medizinischen Zwecke anwenden, bei denen ein sicherer Verschluß eines mittels einer Spritze zu füllenden, unter erhöhtem Druck stehenden Raumes erzielt werden soll. Insbesondere kann dieser Raum ein am vorderen Ende eines Katheterschlauches befindlicher Ballon sein, der über einen zum Katheterschlauch zusätzlichen Kanal mit dem Rückschlagventil verbunden ist und der Festlegung des Katheterschlauches nach dem Einführen in den menschlichen Körper dient. Zum Entleeren dieses Raumes braucht der Schieber 18 lediglich mit dem Mundstück 9 in die Öffnungsstellung gebracht zu werden, worauf die Luft oder das Fluidum entweicht oder mit der Spritze abgesaugt werden kann.

- 1 -

**A 112**

Patentansprüche

1. Rückschlagventil für medizinische Zwecke, insbesondere für Ballonkatheter, bei dem ein hohles Ventilgehäuse an einer Stirnseite eine Anschlußöffnung zum Einführen eines Spritzen-Mundstückes, an der anderen Stirnseite mindestens eine Durchgangsöffnung und in seinem Innenraum nahe der Anschlußöffnung einen ersten Anschlag und, zur gegenüberliegenden Stirnseite hin versetzt, einen zweiten Anschlag für ein Verschlußstück aufweist und das Verschlußstück vom Spritzen-Mundstück gegen eine elastische Kraft aus einer die stirnseitigen Öffnungen gegeneinander sperrenden Schließstellung in eine diese Öffnungen untereinander verbindende Öffnungsstellung verlagerbar ist, dadurch gekennzeichnet, daß der zweite Anschlag (17; 117) am Ventilgehäuse (3; 103) angeformt ist, daß das Verschlußstück ein Schieber (18; 118) ist, der mit seiner Umfangsfläche mindestens eine Ventilöffnung (15; 115) am zylindrischen Innenumfang (14; 114) des Ventilgehäuses übersteuert und daß der erste Anschlag (16; 116) eine so geringe Erhebung hat, daß der Schieber unter Ausnutzung der elastischen Verformbarkeit von Ventilgehäuse und/oder Schieber durch die Anschlußöffnung (6; 106) hindurch einführbar ist.

2. Rückschlagventil nach Anspruch 1, dadurch gekennzeichnet, daß der zweite Anschlag (17) von einer steifen einstückig und gemeinsam mit dem Ventilgehäuse (3) geformten Stirnwand (7) getragen ist.

3. Rückschlagventil nach den Ansprüchen 1 oder 2, dadurch gekennzeichnet, daß der erste Anschlag (16) ein Ringwulst ist.

4. Rückschlagventil nach einem der Ansprüche 1 bis 3, dadurch gekennzeichnet, daß der Schieber (18) mindestens zwei in Axialrichtung gegeneinander versetzte Umfangs-Dichtlippen (19, 20) aufweist.

5. Rückschlagventil nach einem der Ansprüche 1 bis 4, dadurch gekennzeichnet, daß der Schieber (8) zumindest über einen Teil seiner axialen Länge einen mit der Durchgangsöffnung (11) in Verbindung stehenden Hohlraum (21) aufweist.

6. Rückschlagventil nach einem der Ansprüche 1 bis 5, dadurch gekennzeichnet, daß zur Erzeugung der elastischen Kraft eine Schraubenfeder (22), die sich im Bereich des zweiten Anschlags (17) am Ventilgehäuse (3) abstützt, an dem Schieber (18) angreift.

7. Rückschlagventil nach den Ansprüchen 5 und 6, dadurch gekennzeichnet, daß die Schraubenfeder (22) sich in den Hohlraum (21) des Schiebers (18) erstreckt.

8. Rückschlagventil nach einem der Ansprüche 1 bis 7, dadurch gekennzeichnet, daß die mindestens eine Ventilöffnung (15) die Umfangswand (6) des Ventilgehäuses (3) durchsetzt und mit einer von außen abgedeckten auch die Durchgangsöffnung (11) bildenden Nut am Außenumfang des Ventilgehäuses (3) verbunden ist.

9. Rückschlagventil nach einem der Ansprüche 1 bis 7, dadurch gekennzeichnet, daß die mindestens eine Ventilöffnung (115) durch eine Nut (112) am Innenumfang (114) des Ventilgehäuses (103), die eine größere axiale Erstreckung hat als die dichtende Länge des Schiebers (118), gebildet ist.

10. Rückschlagventil nach einem der Ansprüche 1 bis 9, dadurch gekennzeichnet, daß die mindestens eine Ventilöffnung (15; 115) einen größeren axialen Abstand vom ersten Anschlag (16; 116) hat als die dichtende Länge des Schiebers (18; 118).

11. Rückschlagventil nach einem der Ansprüche 1 bis 10, dadurch gekennzeichnet, daß der Außenumfang (2) des Ventilgehäuses (3) zylindrisch ist und einen Durchmesser von 7 mm hat.

Fig.1    Fig.2    Fig.3    Fig.4

1/1

0135140

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 850 176 (GOTTSCHALK) <br> * Spalte 9, Zeilen 17-62 * <br><br> --- | 1,2,8 | A 61 M 25/00 |
| A | US-A-3 131 694 (GARTH) <br> * Insgesamt * <br><br> --- | 1,2,8 | |
| A | US-A-3 366 138 (GRAHAM) <br> * Figur 1 * <br><br> --- | 1,6-8 | |
| A | US-A-3 385 301 (HARAUTUNEIAN) <br> * Spalte 2, Zeile 41 - Spalte 3, Zeile 42 * <br><br> --- | 1,8 | |
| P,A | GB-A-2 127 295 (KENDALL) <br> * Seite 2, Zeilen 17-130 * <br><br> --- | 1,2,8 | |
| P,A | US-A-4 429 856 (JACKSON) <br><br> --- | | **RECHERCHIERTE SACHGEBIETE (Int. Cl.4)** <br><br> A 61 M <br> F 16 K <br> F 16 L |
| D,A | US-A-3 831 629 (MACKAL) <br><br> ----- | | |

Der vorliegende Recherchenbericht wurde für alle Patentansprüche erstellt.

| Recherchenort <br> DEN HAAG | Abschlußdatum der Recherche <br> 15-11-1984 | Prüfer <br> VERELST P.E.J. |
|---|---|---|